# EUROPEAN PATENT SPECIFICATION

(11) **EP 1 995 595 B1**
(45) Date of publication and mention of the grant of the patent: **19.10.2011**
(21) Application number: 07739368.4
(22) Date of filing: 15.03.2007
(51) Int. Cl.: G01N 33/53

(54) **METHOD OF EVALUATING SKIN SENSITIVITY LEVEL BY USING SQUAMOUS CELL CARCINOMA-RELATED ANTIGEN AS INDICATION**
VERFAHREN ZUR BEWERTUNG DES HAUTEMPFINDLICHKEITSNIVEAUS DURCH VERWENDUNG VON MIT PLATTENEPITHELKARZINOM ZUSAMMENHÄNGENDEM ANTIGEN ALS INDIKATION
PROCÉDÉ PERMETTANT D'ÉVALUER LE NIVEAU DE SENSIBILITÉ DE LA PEAU EN UTILISANT COMME INDICATION UN ANTIGENE ASSOCIÉ AU CARCINOME DE CELLULES SQUAMEUSES

(30) Priority: 17.03.2006 JP 2006075024
(43) Date of publication of application: 26.11.2008
(73) Proprietor: Shiseido Company, Limited, Tokyo 104-8010 (JP)
(72) Inventor: KATAGIRI, Chika, c/o SHISEIDO RESEARCH CENTER, Yokohama-shi Kanagawa 236-8643 (JP); NAKANISHI, Jotaro, c/o SHISEIDO RESEARCH CENTER, Yokohama-shi Kanagawa 236-8643 (JP); HIBINO, Toshihiko, c/o SHISEIDO RESEARCH CENTER, Yokohama-shi Kanagawa 236-8643 (JP)
(74) Representative: Ulmann, Catherine Claire
(86) International application number: PCT/JP2007/055926
(87) International publication number: WO 2007/108523

(56) References cited:
- EP-A- 1 860 437
- JP-A- 10 221 340
- JP-A- 2004 028 698
- JP-A- 2005 272 343
- RIVAS M V ET AL: "Identification of Aberrantly Regulated Genes in Diseased Skin Using the cDNA Differential Display Technique" JOURNAL OF INVESTIGATIVE DERMATOLOGY, NATURE PUBLISHING GROUP, GB, vol. 108, no. 2, 1 January 1997 (1997-01-01), pages 188-194, XP003002207 ISSN: 0022-202X
- TAKEDA A ET AL: "Overexpression of serpin squamous cell carcinoma antigens in psoriatic skin" JOURNAL OF INVESTIGATIVE DERMATOLOGY, NATURE PUBLISHING GROUP, GB, vol. 118, no. 1, 1 January 2002 (2002-01-01), pages 147-154, XP003002003 ISSN: 0022-202X
- SULE CATALTEPE ET AL: "Co-expression of the squamous cell carcinoma antigens 1 and 2 in normal adult human tissues and squamous cell carcinomas" JOURNAL OF HISTOCHEMISTRY AND CYTOCHEMISTRY, HISTOCHEMICAL SOCIETY, NEW YORK, NY, US, vol. 48, no. 1, 1 January 2000 (2000-01-01), pages 113-122, XP002974324 ISSN: 0022-1554
- HORIUCHI YASUHIRO ET AL: "Immunohistochemical study of elevated expression of squamous cell carcinoma (SCC)-related antigens in erythrodermic epidermis" JOURNAL OF DERMATOLOGY, JAPANESE DERMATOLOGICAL ASSOCIATION, TOKYO, JP, vol. 21, no. 2, 1 January 1994 (1994-01-01), pages 67-72, XP009115861 ISSN: 0385-2407
- CAMPBELL B ET AL: "Squamous cell carcinoma antigen in patients with cutaneous disorders" JOURNAL OF THE AMERICAN ACADEMY OF DERMATOLOGY, C.V. MOSBY, ST. LOUIS, MO, US, vol. 22, no. 4, 1 April 1990 (1990-04-01), pages 639-642, XP009104761 ISSN: 0190-9622
- DUK J M ET AL: "Elevated levels of squamous cell carcinoma antigen in patients with a benign disease of the skin" CANCER, AMERICAN CANCER SOCIETY, PHILADELPHIA, PA, US, vol. 64, no. 8, 15 October 1989 (1989-10-15), pages 1652-1656, XP009104760 ISSN: 0008-543X
- MITSUISHI K.: 'The squamous cell carcinoma antigens as relevant biomarkers of atopic dermatitis' CLINICAL & EXPERIMENTAL ALLERGY vol. 35, no. 10, 2005, pages 1327 - 1333, XP003017844
- IDEHARA K.: 'Microarray-ho ni yoru Shinki Shindan Marker no Tansaku 1 -Henpei Johi Saibo Gan Kogen no Shindan Marker to Shite no Igi-' DAI 52 KAI JAPANESE SOCIETY OF LABORATORY MEDICINE SOAKI . DAI 45 KAI JAPAN SOCIETY OF CLINICAL CHEMISTRY NENKAI RENGO TAIKAI SHOROKUSHU vol. S1-3, 2005, page 23, XP003017845

## Description

### TECHNICAL FIELD

The present invention provides a method of evaluating the degree of skin sensitivity using cellular squamous cell carcinoma antigen (SCCA) as an indicator thereof.

### BACKGROUND ART

The number of people who think they have sensitive skin, namely highly sensitive skin exhibiting strong reactivity to external irritation and stress, has tended to increase in recent years. In particular, more than 70% of young women in their twenties and thirties replied to a survey that they have sensitive skin. Factors responsible for sensitive skin include a decrease in the barrier function of skin, a decrease in the skin's irritation threshold, drying of the skin, inflammatory substances of contact dermatitis, physicochemical irritation, stress, physical condition, seasonal changes, ultraviolet rays and menstruation. In addition, there were also cases in which respondents indicated that they have sensitive skin despite being resistant to stress on the skin, thus resulting in the need for a suitable and objective biochemical indicator of skin sensitivity.

This type of sensitive skin is defined in the manner indicated below.
1. Skin susceptible to the occurrence of skin problems due to specifically reacting to substances such as externally applied pharmaceuticals, cosmetics, plants, ultraviolet rays or metals and the like on a regular basis, and which is constitutionally sensitive to allergens (such as pollen or perfume) or irritants (such as alcohol) due to a decrease in the barrier function of the skin, and more specifically, is considered to be the result of an allergic constitution or hypersensitive constitution, presenting with symptoms such as susceptibility to dry skin (rough skin), susceptibility to chapped skin and susceptibility to rashes due to a decrease in the skin's barrier function.
2. Skin susceptible to temporary skin problems during times when the inherent resistance of the skin or physiological functions of skin are weakened by such factors as a lack of sleep, fatigue, menstruation, seasonal changes and psychological stress.

The sensitivity of the skin to various external irritation and stress varies from person to person, and for example, even though one person may develop rough skin by sensitively reacting to a certain drug, another person may not exhibit any reaction whatsoever. In addition, although one person may immediately exhibit rough skin in response to a drug or external irritation, another person may only develop rough skin following continuous irritation. In the past, the degree of skin sensitivity was only determined after rough skin had developed. However, once rough skin has developed, there is both a considerable physical and psychological burden, more time is required for recovery or scars may result that are difficult to treat. Accordingly, if it were possible to measure and recognize in advance the degree of sensitivity and reactivity to external irritation and stress of an individual's skin, it would be possible to prevent rough skin in advance, and also would be possible to select skin care that is appropriate for highly sensitive skin, thereby making this extremely important.

Squamous cell carcinoma antigen (SCCA) is an antigen extracted from squamous cell carcinoma cells that exhibits high concentrations in the blood in squamous cell carcinoma of the neck of the cervix, lungs, esophagus and skin, and is frequently used to diagnose squamous cell carcinoma (H. Kato, et al., Cancer 40: 1621-1628 (1997); N. Mino, et al., Cancer 62: 730-734 (1988)). In particular, since levels of SCCA in the blood closely correlate with the stage of advancement, malignancy and tumor size of squamous cell carcinoma, it is a particularly useful cancer marker not only for early cancer detection, but also for evaluation of the efficacy of cancer therapy and diagnosis of the risk of recurrence.

SCCA is also known to demonstrate increased expression in the upper layer of psoriatic epidermis (Takeda, A. et al., J. Invest. Dermatol. (2002) 118(1), 147-154). Psoriasis is a type of skin disease that presents with chronic and recurrent inflammatory parakeratosis characterized by proliferation and differentiation abnormalities of epidermal cells and inflammatory cell infiltration. Psoriasis is thought to occur due to involvement by various environmental factors in addition to genetic factors (Hopso-Havu et al., British Journal of Dermatology (1983) 109, 77-85).

SCCA is encoded by two genes, i.e., SCCA-1 and SCCA-2 genes arranged in tandem on chromosome 18q21.3. Each of the proteins SCCA-1 and SCCA-2 encoded thereby both have molecular weights of about 45000, exhibit a high degree of homology and their homology at the nucleic acid level is 95%. These SCCA proteins belong to a family of ovalbumin-serine protease inhibitors (ov-serpins). Ov-serpins have unique characteristics within the serpin super family. Although serpins are generally said to be excreted and function extracellulary, ov-serpins mainly function within cells in the form of protease inhibitors.

Although SCCA-1 is a papain-like cysteine protease inhibitor, SCCA-2 is a chymotrypsin-like serine protease inhibitor, and despite having high homology, since the amino acid sequence at the reactive site differs from SCCA-1, it has different properties therefrom (Schick et al., J. Biol. Chem. (1997) 27213, 1849-55). Although SCCA-1 and SCCA-2 are known to be expressed at high levels in response to diseases such as psoriasis as well as UV irradiated skin, the manner in which they are involved in skin properties is unknown.

The identification of aberrantly regulated genes in diseased skin, in particular psoriasis, using the cDNA differential display technique, is disclosed in M. Rivas et al., J. Investigative Dermatology, 1997, 108, 188-194.

### DISCLOSURE OF THE INVENTION

As a result of conducting research for the purpose of elucidating the physiological mechanism of the involvement of SCCA in the epidermis, the inventors of the present invention found that the degree of sensitivity and reactivity of skin is related to the amount of SCCA in the epidermis, and that subjects having higher levels of SCCA in the epidermis demonstrated high reactivity to skin irritation. Thus, expression of SCCA was considered to be an indicator of the skin's sensitivity and reactivity, thereby leading to completion of the present invention.

The present invention provides a method of evaluating the degree of skin sensitivity and reactivity on the basis of the expression of squamous cell carcinoma antigen (SCCA), more specifically SCCA-1 and/or SCCA-2, and particularly SCAA-1, in skin corneocytes. Preferably, the expression of SCCA is carried out by an enzyme-linked immunosorbent assay (ELISA) using antibodies specific to SCCA. In a more preferable aspect thereof, a sample of the skin horny layer is harvested by tape stripping.

According to the method of the present invention, the degree of skin sensitivity and reactivity can be evaluated at the biochemical level.

### BRIEF DESCRIPTION OF THE DRAWINGS

Fig. 1 shows correlation diagrams between expressed amounts of SCCA and TEWL in normal skin.
Fig. 2 shows TEWL values and expressed amount of SCCA-1 following application of oleic acid.
Fig. 3 shows fluctuations in TEWL and expressed amount of SCCA-1 following application of oleic acid.
Fig. 4 shows a correlation diagram between fluctuations in TEWL and expressed amount of SCCA-1.

### BEST MODE FOR CARRYING OUT THE INVENTION

As mentioned above, SCCA is a protein having a molecular weight of about 45,000 present in squamous cell carcinoma cells and psoriatic epidermis. The amino acid sequences of SCCA-1 and SCCA-2 along with the nucleic acid sequences by which they are encoded are described in Takeda A. et al., J. Invest. Dermatol. 118, 147-154 (2002) (op. cit.).

Measurement of expression of SCCA in accordance with the present invention can be carried out quantitatively or qualitatively by any method capable of measuring SCCA. Specific examples of which include various methods such as an immunoassay using an antibody specific to SCCA, for example, ELISA using an enzyme label, RIA using a radioactive label, immunonephelometry, western blotting, latex agglutination or erythrocyte agglutination. Examples of immunoassays include competitive assays and sandwich assays. In addition, the amount of the expressed SCCA can also be determined by measuring the amount of the intracellulary-expressed gene coding for SCCA. In this case, the expression of SCCA is preferably determined by measuring the amount of the intracellular mRNA coding for SCCA. Extraction of mRNA and quantitative or qualitative measurement of the amount thereof are commonly known in the art, and can be carried out by various known methods such as PCR, 3SR, NASBA or TMA. In addition, expression of SCCA can also be qualitatively determined through in situ hybridization or measurement of the biological activity thereof.

Although harvesting of specimens in the form of skin horny layer samples can be carried out by any method, tape stripping is preferable from the viewpoint of the ease of the procedure. Tape stripping refers to a method for harvesting horny layer samples by applying a pressure-sensitive adhesive tape to the skin epidermis, and then peeling off the tape, and thus rendering the skin horny layer to be adhered to the peeled pressure-sensitive adhesive tape. Use of the tape stripping method makes it possible to evaluate sensitive skin in a non-invasive manner using SCCA as an indicator by allowing expression of SCCA to be measured simply by harvesting the horny layer on a piece of tape. A preferable method for tape stripping comprises, first removing sebaceous matter, dirt and the like from the skin epidermis by cleaning with ethanol or the like, gently pressing a piece of pressure-sensitive adhesive tape cut to a suitable size (for example, 2 x 5 cm) onto the surface of the skin, evenly pressing the tape onto the skin while applying equal force to the entire piece of tape, and then peeling off the pressure-sensitive adhesive tape with uniform force. The pressure-sensitive adhesive tape may be commercially available cellophane tape and the like, and examples of such tape that can be used include Scotch Super Strength Mailing Tape (3M Corp.) and Cellophane Tape (Cellotape (registered trademark), Nichiban Co., Ltd.). SCCA present in a skin horny layer sample adhered to the pressure-sensitive adhesive tape can be isolated and extracted from the tape by immersing the piece of tape in a suitable extraction solution such as Tris-buffer (pH 8.0) (0.1 M Tris-HCl, 0.14 M NaCl, 0.1% Tween-20) and extracting the horny layer.

In a preferable embodiment of the present invention, SCCA is measured by an immunoassay method such as ELISA. The antibody specific to SCCA used in ELISA may be a monoclonal antibody or polyclonal antibody. Methods for preparing monoclonal antibody and polyclonal antibody are commonly known among persons with ordinary skill in the art, and are described in, for example, Lunstrum et al., J. Biol. Chem. 1986, 261: 9042-9048; Hurle et al., J. Cell. Science 1994, 107: 2623-2634.

In the method as claimed in the present invention, a sandwich immunoassay method is particularly preferable. The sandwich immunoassay can be carried out, for example, in a manner described below.

One of two antibodies specific to SCCA is immobilized on a support as the first antibody. A solid support is preferable for the support, and any support conventionally used in immunoassays as a solid support, for example, may be used, examples of which include a polymer support made of styrene or polystyrene formed to an arbitrary size and shape, and a reaction vessel molded from these suitable materials such as the inner walls of the wells of an ELISA plate.

Immobilization of the first antibody to the support can be carried out in accordance with conventional methods. For example, the first antibody can be dissolved in, for example, phosphate-buffered saline (PBS) or borate buffer, followed by adsorbing to a support to immobilize the first antibody to the support. Moreover, blocking is preferably carried out to inhibit non-specific binding by adding a suitable blocking agent such as PBS-BSA or a commercially available blocking agent such as Block Ace (Dainippon Pharmaceutical Co., Ltd.) to the support immobilized with the first antibody in this manner, followed by incubating for 5 minutes to several days, preferably 10 minutes to 24 hours and more preferably for 10 minutes to 3 hours at about 4 to 40°C and preferably 20 to 37°C.

The other one of the two antibodies specific to SCCA is used as the secondary antibody and labeled. Examples of labels include enzyme labels, radioactive labels, fluorescent labels and the like. In the case of enzyme labeling, the enzyme can be bound directly to the secondary antibody, or the secondary antibody can be labeled with the enzyme through interactive proteins such as avidin-biotin. Binding of an enzyme to the antibody can be carried out by, for example, respectively introducing a thiol group into enzyme and antibody to be labeled by using a commercially available thiol group insertion reagent and then binding the two via S-S bonds. Examples of enzymes include horseradish peroxidase, alkaline phosphatase and β-D-galactosidase. Enzyme can be detected using a substrate specific to the enzyme. For example, in the case of using horseradish peroxidase, TMB (3,3',5,5'- tetramethylbenzindine) or ABTS (2,2'-azine-di[3- ethylbenzthiazoline sulfonate]) can be used.

This immunoassay method comprises the step of incubating a mixture of the support immobilized with the first antibody, the labeled secondary antibody and the test sample, thereby rendering the SCCA in the test sample to bind to the first antibody immobilized on the support and rendering the labeled secondary antibody to bind to the SCCA molecules.

In this manner, the labeled antibody is immobilized on the support through the first antibody immobilized on the support and SCCA derived from the sample in an amount that reflects the amount of SCCA in the sample. The incubation is carried out in a suitable buffer such as PBS for 5 minutes to several days, preferably from 10 minutes to 24 hours and more preferably from 10 minutes to 3 hours at about 4 to 40°C and preferably 20 to 37°C.

Next, a procedure is carried out for separating unbound labeled antibody from the support. In the case of using a solid support for the support, this separation procedure can be carried out easily by solid-liquid separation. In the case of using a fixed, known amount of the labeled secondary antibody, the label bound to the support, unbound label or both can be measured. On the other hand, in the case of using a labeled antibody, the label bound to the support is detected and measured. Detection of the label bound to the support is preferably carried out by first washing the support with a washing solution, for example, a buffer containing a suitable surfactant such as PBS-Tween-20 to remove unbound labeled antibody. Detection can be carried out in accordance with conventional methods depending on the type of label.

The following provides a more detailed explanation of the present invention through specific embodiments thereof. However, the present invention is not limited to these examples.

### Materials and Methods

### (1) Antibody

Polyclonal antibody recognizing both SCCA-1 and SCCA-2 was prepared using SCCA (SCCA-1 and SCCA-2) purified from scales of psoriatic epidermis. After centrifuging a scale extract of psoriatic epidermis (extraction solution: 0.1 M Tris-HCl (pH 8.0), 0.14 M NaCl), the supernatant was purified with Sephacryl S-200, DEAE Sepharose, Mono Q, Mono S, Mono P and Superrose 6, followed by using the purified supernatant as antigen and using rabbits for the sensitized animals.

Anti-SCCA-1 monoclonal antibody and anti-SCCA-2 monoclonal antibody were acquired from Santa Cruz Biotechnology, CA, USA.

### (2) ELISA

Skin horny layer samples were harvested by tape stripping comprising the steps of applying a transparent pressure-sensitive adhesive tape (Cellotape (registered trademark), Nichiban Co., Ltd.) to the surface of the skin followed by peeling off the tape. The tape adhered with skin horny layer was cut into pieces, immersed in an extraction buffer (0.1 M Tris-HCl (pH 8.0), 0.14 M NaCl, 0.1% Tween-20, 1 ml) and subjected to ultrasonic treatment (20 sec x 4) to prepare sample extracts.

100 µl aliquots of polyclonal anti-SCCA antibody diluted with PBS (1:1000 dilution) were dispensed into each well of a 96-well ELISA plate and allowed to stand overnight at room temperature to bind to the solid phase of the plate. Subsequently, in order to inhibit non-specific binding to the plate, the bound polyclonal antibody was incubated for 1 hour in a blocking solution (solution containing Block Ace diluted with PBS-Tween-20, 300 µl/well).

50 µl of the above sample extract were added to each well of the ELISA plate and allowed to react for 2 hours at 37°C. Monoclonal anti-SCCA-1 antibody (diluted to 1:1000) was then added and allowed to react for 1 hour at 37°C. Next, secondary antibody in the form of horseradish peroxidase-labeled anti-mouse antibody was added and allowed to react for 1 hour at 37°C. After washing with 0.1% Tween-20 PBS, a substrate, i.e., 3,3',5,5'-tetramethylbenzindine (TMB) was added followed by color development using the TMB Peroxidase ELISA Substrate Kit (Bio-Rad) and measuring at 630 nm.

### (3) Measurement of Skin Transepidermal Water Loss (TEWL)

The TEWL of specimens (from the face and inner arm of normal subjects) was measured using a TEWAmeter (TM120).

### (4) Correlation Between SCCA Expression and Transepidermal Water Loss (TEWL)

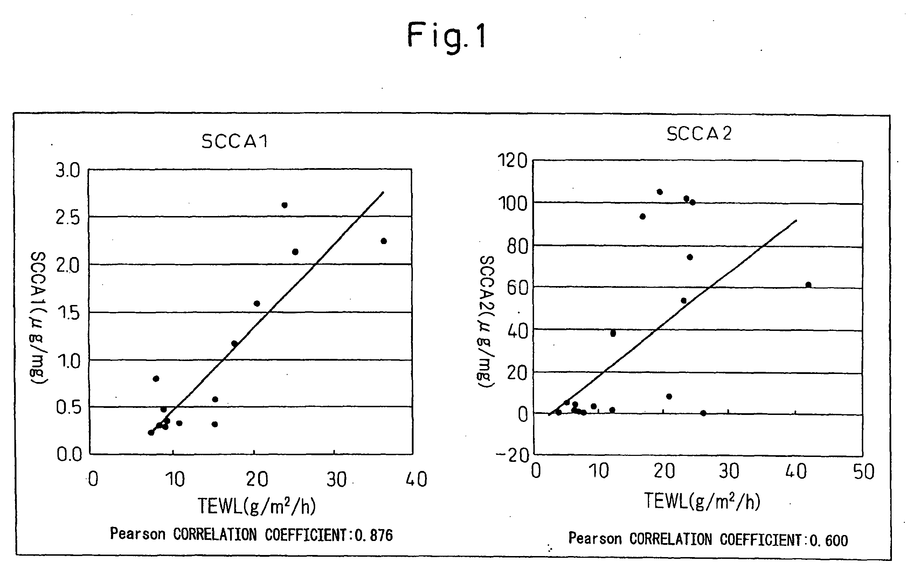

The amount of SCCA expressed in the skin horny layer was measured by ELISA together with investigating a skin physiological parameter in the form of TEWL to investigate the correlation between SCCA expression and TEWL. Those results are shown in Fig. 1. The Pearson correlation coefficient between SCCA-1 and TEWL was 0.876, while that between SCCA-2 and TEWL was 0.600, with a significant correlation being observed for both. Thus, the expressed amounts of both SCCA-1 and SCCA-2, and particularly SCCA-1, were found to increase when TEWL is high and skin condition is comparatively poor.

### (5) SCCA Expression During Chemical Irritation

A chemical irritant in the form of 10% oleic acid was applied to skin of the forehead (exposed area) and inner arm (unexposed area) on days 1 and 2. In addition to measuring TEWL values of the skin prior to application of oleic acid and on day 4 following application of oleic acid, the horny layer was harvested from each area of skin by tape stripping followed by measurement of the expressed amounts of SCCA-1 by ELISA. The relationship was then investigated between TEWL and skin before and after application of oleic acid.

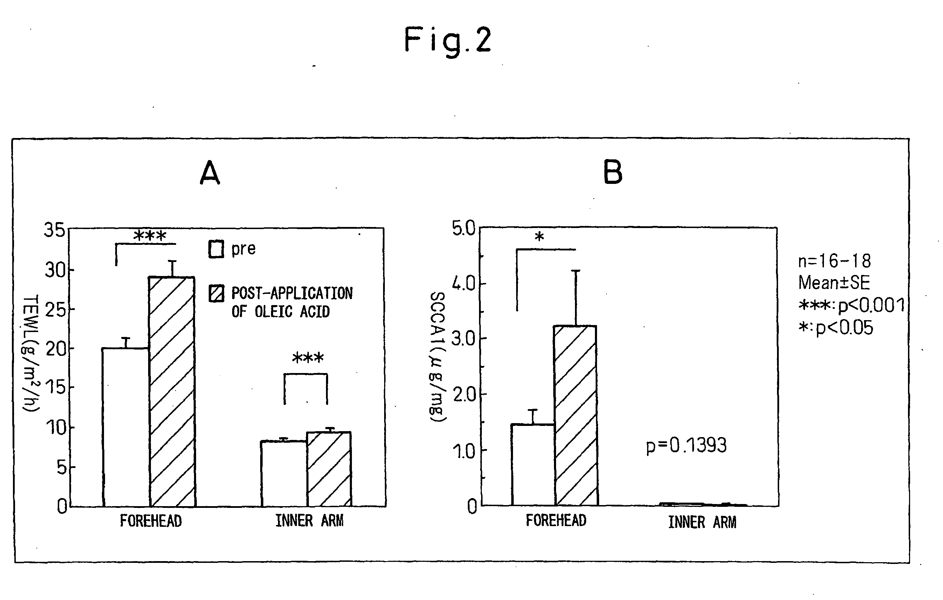

Fig. 2 shows the relationship between TEWL and the skin prior to application of oleic acid (a) and the relationship between expression of SCCA-1 and the skin prior to application of oleic acid (b). As is clear from Fig. 2(a), in contrast to having observed a significant increase in TEWL values on the forehead due to the application of oleic acid, on the inner arm, significant increases in TEWL were not observed attributable to chemical irritation. Thus, although the application of oleic acid on the forehead caused a significant decrease in the barrier function of the skin, there was found to be hardly any decrease in barrier function on the inner arm, thus demonstrating that the forehead is more sensitive to chemical irritation than the inner arm.

Fig. 2(b) shows the expressed amounts of SCCA-1 before and after application of oleic acid. On the basis of these results, the amount of SCCA-1 on the forehead is high, and the amount of SCCA-1 increases significantly in response to chemical irritation. On the other hand, the amount of SCCA-1 on the inner arm was considerably lower than that on the face. Thus, the forehead, which is more sensitive to chemical irritation, was clearly demonstrated to exhibit higher expression of SCCA-1 as compared with the inner arm.

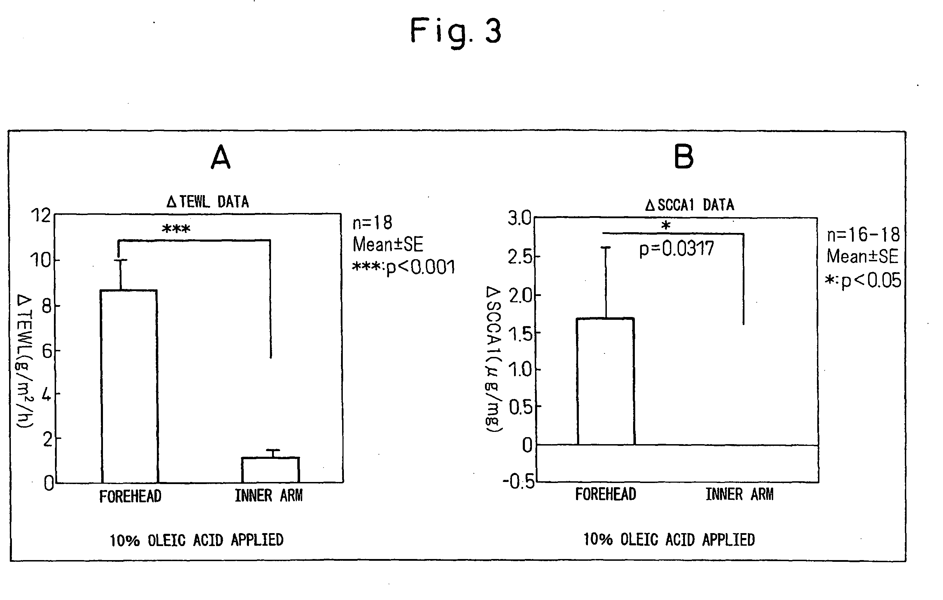

Fig. 3 shows fluctuations in TEWL values (a) and fluctuations in expression of SCCA-1 (b) on the forehead and inner arm before and after application of oleic acid (a). Although Fig. 3(a) indicates the forehead to be more sensitive to chemical irritation than the inner arm in the same manner as Fig. 2(a), in contrast to Fig. 3(b) indicating a significant increase in expression of SCCA-1 due to chemical irritation on the forehead, which was previously demonstrated to be highly sensitive, expression of SCCA-1 on the inner arm, which was previously demonstrated to have low sensitivity, was not observed to demonstrate a significant change. Thus, the forehead, which is highly sensitive to chemical irritation, demonstrated increased expression of SCCA-1 following application of oleic acid, and this is thought to have led to a decrease in the barrier function of the skin.

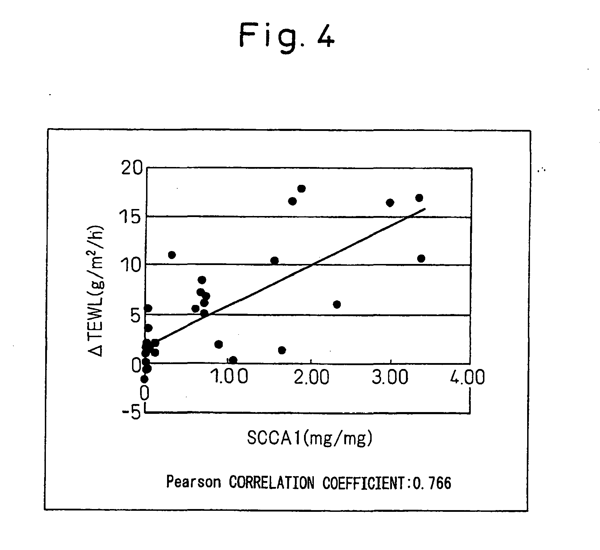

Fig. 4 shows the correlation between fluctuations in TEWL values and fluctuations in expressed amounts of SCCA-1 for skin to which oleic acid was applied. Fig. 4 summarizes values obtained for skin on the forehead and inner arm. It can be seen from this figure that the higher the level of SCCA-1 in the epidermis of an individual prior to application of oleic acid, the greater the fluctuations in TEWL values following application of oleic acid. Namely, this suggested that the higher the level of SCCA-1 in the epidermis of an individual prior to application of oleic acid, the higher the proportion of the decrease in the barrier function of the skin attributable to external irritation (the Pearson correlation coefficient between SCCA-1 and TEWL was 0.7668, thus indicating a significant correlation). Thus, the expression level of SCCA-1 in the epidermis of each individual was suggested to be an indicator of the sensitivity and reactivity of the skin to external irritation in the form of chemical irritation.

### (6) Expression Levels of SCCA-1 in Exposed Skin Presenting with Parakeratosis, Allergic Skin, Atopic Dry Skin and Psoriatic Skin

Horny layer specimens were harvested by tape stripping from an unexposed area of the skin (inner arm) demonstrating normal skin properties, an exposed area of the skin (face) presenting with parakeratosis, skin from patients suffering from rough skin caused by allergic skin attributable to pollenosis, skin from patients suffering from atopic dermatitis, and skin from patients suffering from psoriasis, followed by measurement of expressed amounts of SCCA-1 by ELISA as previously described.

The results are shown in Table 1. Considerable increases in SCCA-1 levels were observed in all skin examined with the exception of the skin from an unexposed site demonstrating normal skin properties (control). Expression levels of SCCA-1 were 16 times higher in atopic dry skin, 90 times higher in exposed skin, 232 times higher in pollenosis-induced allergic skin, and 466 times higher in psoriatic skin as compared with the control. On the basis of these results, SCCA-1 was clearly observed to demonstrate considerably increased expression in skin highly sensitive to irritation, namely in sensitive and highly reactive skin susceptible to further skin roughness at low levels of irritation.

**Table 1 Increases in SCCA-1 in Parakeratotic Skin**

| Skin | SCCA-1 | Statistical Analysis |
|---|---|---|
| Normal skin (unexposed area) | 1.0±0.1 | -- |
| Atopic dry skin | 15.5±4.1 | ** |
| Exposed skin | 90.0±16.6 | *** |
| Allergic skin | 232.2±32.6 | *** |
| Psoriasis | 465.8±146.0 | *** |

| | | |
|---|---|---|
| Mean±SD: **: p<0.01, ***: p<0.001 (t-test) | | |

## Claims

1. An in vitro method for evaluating the degree of skin sensitivity comprising: using the expression of squamous cell carcinoma antigen (SCCA) in skin corneocytes as an indicator thereof, wherein a higher level of SCCA in the epidermis demonstrates high reactivity to skin irritation.

2. The method according to claim 1, wherein the sensitivity of the skin is that to chemical irritation.

3. The method according to claim 1 or claim 2, wherein the expression of SCCA is carried out by an enzyme-linked immunosorbent assay (ELISA) using antibodies specific to SCCA.

4. The method according to any of claims 1 to 3, wherein the skin horny layer specimen is harvested by tape stripping.

5. The method according to any of claims 1 to 4, wherein the SCCA is SCCA-1.

## Patentansprüche

1. In-vitro-Verfahren zur Beurteilung des Hautempfindlichkeitsgrades, umfassend die Verwendung der Expression des Plattenepithelkarzinom-Antigens (Squamous Cell Carcinoma Antigen, SCCA) in Haut-Corneocyten als Indikator hierfür, wobei eine höhere Konzentration von SCCA in der Epidermis eine hohe Reaktivität gegenüber einer Hautreizung belegt.

2. Verfahren nach Anspruch 1, wobei die Empfindlichkeit der Haut die gegenüber einer Chemikalien-Reizung ist.

3. Verfahren nach Anspruch 1 oder Anspruch 2, wobei die Expression von SCCA durch einen enzymgekoppelten Immunsorptionstest (ELISA) unter Verwendung von für SCCA spezifischen Antikörpern ermittelt wird.

4. Verfahren nach einem der Ansprüche 1 bis 3, wobei die Hauthornschichtprobe durch Klebebandablösung gewonnen wird.

5. Verfahren nach einem der Ansprüche 1 bis 4, wobei das SCCA SCCA-1 ist.

## Revendications

1. Procédé in vitro pour l'évaluation du degré de sensibilité de la peau comprenant le fait d'utiliser l'expression de l'antigène du carcinome à cellules squameuses (SCCA) dans des cornéocytes de la peau comme un indicateur de celle-ci, où un niveau supérieur de SCCA dans l'épiderme démontre une forte réactivité à une irritation cutanée.

2. Procédé selon la revendication 1, dans lequel la sensibilité de la peau est celle due à une irritation chimique.

3. Procédé selon la revendication 1 ou la revendication 2, dans lequel l'expression du SCCA est effectuée par un essai d'immuno-absorption par enzyme liée (ELISA) utilisant des anticorps spécifiques du SCCA.

4. Procédé selon l'une quelconque des revendications 1 à 3, dans lequel le spécimen de couche cornée de la peau est récolté par décapage par adhésifs.

5. Procédé selon l'une quelconque des revendications 1 à 4, dans lequel le SCCA est SCCA-1.
